# EUROPÄISCHE PATENTSCHRIFT

(11) **EP 2 361 592 B1**
(45) Veröffentlichungstag und Bekanntmachung des Hinweises auf die Patenterteilung: **02.03.2016**
(21) Anmeldenummer: 10015593.6
(22) Anmeldetag: 14.12.2010
(51) Int. Cl.: A61F 2/38, A61F 2/00, A61F 2/30

(54) **Kniegelenk-Endoprothese mit einem Zwischenelement mit Ausgleichelement**
Knee joint endoprosthetic with an intermediate element with compensating element
Endoprothèse de genouillère dotée d'un élément intermédiaire doté d'un élément d'égalisation

(30) Priorität: 19.02.2010 DE 102010008620
(43) Veröffentlichungstag der Anmeldung: 31.08.2011
(73) Patentinhaber: Mathys AG Bettlach, 2544 Bettlach (CH)
(72) Erfinder: Frankhauser, Christoph, 4500 Solothurn (CH); Grunder, Beat, 3076 Worb (CH); Supper, Walter, 2540 Worb (CH)
(74) Vertreter: Körfer, Thomas

(56) Entgegenhaltungen:
- EP-A1- 0 611 559
- WO-A2-02/078565
- WO-A2-2008/048819
- US-A- 6 045 581

## Beschreibung

Die Erfindung bezieht sich auf eine Kniegelenk-Endoprothese zum Ausbilden einer Gelenkverbindung zwischen einem Femurknochen und einem Tibiaknochen, die aus einer Femurkomponente, einer Tibiakomponente sowie einem Zwischenelement besteht.

Als künstlicher Ersatz für ein Kniegelenk wird häufig eine dreiteilige Kniegelenk-Endoprothese verwendet. Die EP 0 611 559 A1 beschreibt zum Beispiel eine dreiteilige unikondyläre Knieprothese. Eine Femurkomponente wird dabei mit dem Femurknochen verbunden, eine Tibiakomponente ist mit dem Tibiaknochen verschraubt oder mittels Knochenzement verbunden. Die der Tibiakomponente abgewandte Seite des Zwischenelements bildet eine Gleitfläche, auf der die Femurkomponente abrollt. Das Zwischenelement wird durch eine Schnappverbindung in der Tibiakomponente fixiert. Dazu weist ein tibiaseitiger Bereich des Zwischenelements eine kleinere Außenkontur als ein femurseitiger Bereich des Zwischenelements auf. An der anterioren und posterioren Seite des tibiaseitigen Bereichs des Zwischenelements ist jeweils ein Vorsprung ausgebildet, der in eine entsprechende Nut in der Tibiakomponente einrastet. Die WO 2008/048819 beschreibt ebenfalls eine Knieprothese mit Zwischenelement. Die WO 02/078565 zeigt die Merkmale des Oberbegriffs von Anspruch 1.

Bei der Verbindung zwischen Tibiakomponente und Zwischenelement sind nur geringe Toleranzen erlaubt, damit das Zwischenelement gut und verrutschsicher in die Tibiakomponente einschnappt und dort ohne Spiel integriert wird. Das Zwischenelement besteht häufig aus Polyethylen, insbesondere einem ultrahochmolekularen Polyethylen UHMWPE, das sich durch geringen Abrieb auszeichnet. Das Zwischenelement wird mit Gammastrahlung sterilisiert und bis zum klinischen Einsatz in einer sterilen Verpackung aufbewahrt. Nach der Gammasterilisation kommt es im Laufe der Zeit zu einem geringfügigen volumetrischen Schrumpfen des Zwischenelements. Daraus ergibt sich der Nachteil, dass die engen Toleranzen in der Abmessung nicht mehr erfüllt werden. Dies führt zu einem Bewegungsfreiraum zwischen der Tibiakomponente und dem Zwischenelement und in Folge dessen zu einem schnelleren Verschleiß der beiden Komponenten. Außerdem führt dies zu einem ungenauen Gleitverhalten zwischen Femurkomponente und Zwischenelement.

Es ist somit die Aufgabe der vorliegenden Erfindung, eine Kniegelenk-Endoprothese zu beschreiben, die über lange Zeit fest und ohne Spiel in der Tibiakomponente fixierbar ist bzw. fixiert bleibt.

Die Aufgabe wird durch die erfindungsgemäße Kniegelenk-Endoprothese gemäß Anspruch 1 gelöst. In den Unteransprüchen sind vorteilhafte Weiterbildungen der erfindungsgemäßen Kniegelenk-Endoprothese dargestellt.

Die erfindungsgemäße Kniegelenk-Endoprothese zum Ausbilden einer Gelenkverbindung zwischen einem Femurknochen und einem Tibiaknochen umfasst eine Femurkomponente, die mit dem Femurknochen verbindbar ist, eine Tibiakomponente, die mit dem Tibiaknochen verbindbar ist und ein Zwischenelement, das zwischen der Tibiakomponente und der Femurkomponente angeordnet ist. Dabei weist das Zwischenelement ein Ausgleichselement auf. Das Zwischenelement mit dem Ausgleichselement ist unter einer Spannung in die Tibiakomponente eingepresst. Bei einem Schrumpfen des Zwischenelements liefert das Ausgleichselement genügend Volumen, sodass das Zwischenelement auch bei einer längeren Lagerung vor dem klinischen Einsatz und während der Verweildauer im Patienten ohne Spiel in der Tibiakomponente sitzt.

Erfindungsgemäß weist das Zwischenelement mindestens eine Ausnehmung im Bereich des Ausgleichselements auf. Das Material, aus dem ein Zwischenelement besteht, besitzt typischerweise eine geringe Kompressibilität. Beim Einbringen des Zwischenelements in die Tibiakomponente wirkt insbesondere im Bereich des Ausgleichselements ein erhöhter Druck, unter dem das Material in die Mehrzahl von Ausnehmung hinein ausweicht. Die Mehrzahl von Ausnehmung erhöhen somit die Kompressibilität des Zwischenelements und verhindern eine Beschädigung des Zwischenelements.

Es ist besonders vorteilhaft, wenn das Zwischenelement auf einer an die Tibiakomponente angrenzenden Seite mindestens einen Vorsprung aufweist, wobei der Vorsprung in eine Nut in der Tibiakomponente eingreift und das Ausgleichselement in den Vorsprung integriert ist. Das Ausgleichselement ist in besonders vorteilhafter Weise als eine vorspringende Lippe an dem Vorsprung des Zwischenelements ausgebildet. Dadurch wird auch bei Schwund des Materials des Zwischenelements der passgenaue Sitz in der Tibiakomponente garantiert. Die vorspringende Lippe kann durch einfache Änderungen der Formteile in der Fertigung hergestellt werden. Des Weiteren können dadurch auch etwas Fertigungstoleranzen für das Zwischenelement ausgeglichen werden. Dies führt zu einem geringeren Ausschuss an Zwischenelementen in der Produktion und erlaubt eine kostengünstigere Herstellung.

Eine weitere vorteilhafte Ausführung der Kniegelenk-Endoprothese weist das Zwischenelement eine Nut auf, in die ein Vorsprung in der Tibiakomponente eingreift, wobei das Ausgleichselement im Bereich der Nut im Zwischenelement ausgebildet ist. Dies erlaubt eine kompaktere Form des Ausgleichselements, die weniger empfindlich gegen Risse und gegen Abbrechen ist.

Vorteilhafterweise geht die vorspringende Lippe mit abgeflachten Seiten in den Vorsprung des Zwischenelements über. Dadurch wird die vorspringende Lippe stabiler und bröckelt oder bricht beim Einpressen in die Tibiakomponente nicht ab.

Vorteilhafterweise ist die Ausnehmung als Langloch ausgebildet. Dadurch entsteht ein großes Volumen, in das sich das Ausgleichselement leicht hinein bewegen kann.

Von Vorteil ist es ebenso, wenn das Zwischenelement eine Mehrzahl von Ausnehmungen im Bereich des Ausgleichselements aufweist. Diese Ausnehmungen sind dabei mit einer kreisrunden, ovalen und/oder eckigen Kontur ausgebildet. Durch die Mehrzahl von Ausnehmungen wird der Druck im Material des Zwischenelements auf die Mehrzahl von Wandungen zwischen den Ausnehmungen verteilt und ist somit weniger anfällig für Risse oder Materialbruch.

Ausführungsbeispiele der erfindungsgemäßen Kniegelenk-Endoprothese sind in der Zeichnung beispielhaft dargestellt und werden anhand der nachfolgenden Beschreibung näher erläutert. Es zeigen:
- Fig. 1: eine Ausführungsbeispiel einer dreiteiligen unikondylären Kniegelenk-Endoprothese in perspektivischer Ansicht;
- Fig. 2a: ein erstes Ausführungsbeispiel eines Zwischenelements der erfindungsgemäßen Knie-Endoprothese in Seitenansicht;
- Fig. 2b: ein zweites Ausführungsbeispiel des Zwischenelements der erfindungsgemäßen Knie-Endoprothese in Seitenansicht;
- Fig. 3: ein erstes Ausführungsbeispiel einer Tibiakomponente der erfindungsgemäßen Knie-Endoprothese in Seitenansicht;
- Fig. 4a: ein drittes Ausführungsbeispiel des Zwischenelements der erfindungsgemäßen Knie-Endoprothese in Draufsicht;
- Fig. 4b: das viertes Ausführungsbeispiel des Zwischenelements der erfindungsgemäßen Knie-Endoprothese in Draufsicht und
- Fig. 4c: ein fünftes Ausführungsbeispiel des Zwischenelements der erfindungsgemäßen Knie-Endoprothese in Draufsicht.

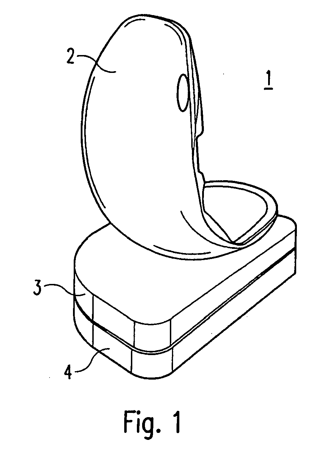

Fig. 1 zeigt eine dreiteilige unikondylären Kniegelenk-Prothese 1. Einander entsprechende Teile sind in allen Figuren mit den gleichen Bezugszeichen versehen. Sie besteht aus einer Femur-Komponente 2, die mit dem Femurknochen verbunden ist. Eine Tibiakomponente 4 ist am proximalen Ende des Tibiaknochens fixiert. Ein Zwischenelement 3 ist zwischen der Femurkomponente 2 und der Tibiakomponente 4 eingebracht und bildet eine Artikulationsfläche mit der Femurkomponente.

Als Werkstoff für die Tibiakomponente 4 wird häufig eine Metalllegierungen, insbesondere eine Kobaltbasislegierung, verwendet, in das ein Zwischenelement 3 aus UHMWPE mittels Schnappverbindung fixiert wird. Vor dem klinischen Einsatz wird das Zwischenelement mittels Gammastrahlung sterilisiert. Dies führt nachweislich zu einem Schwund im Volumen des Zwischenelements, sodass auch bei einer passgenauen Fertigung des Zwischenelements 3 dieses nach der Gammasterilierung nicht mehr bündig in die Tibiakomponente 4 sitzt, sodass ein Bewegungsraum zwischen Zwischenelement 3 und Tibiakomponente 4 entsteht.

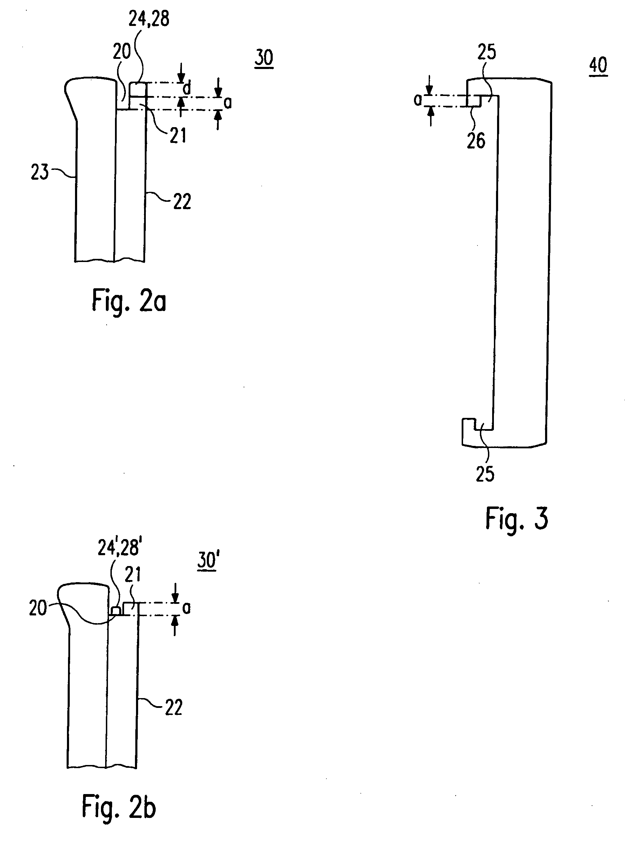

Fig. 2a stellt ein erstes Ausführungsbeispiel eines erfindungsgemäßen Zwischenelements 30 in Seitenansicht dar, das ein Ausgleichselement 24 auf einem Vorsprung 21 im tibiaseitigen Bereich 22 des Zwischenelements 30 aufweist. Die Höhe a des Vorsprungs stimmt in etwa mit der Tiefe einer Nut 25 der Tibiakomponente 40, die in Fig. 3 dargestellt ist, überein. Auf dem Vorsprung 21 ist eine vorspringende Lippe 28 ausgebildet. Die Höhe d der Lippe 28 reicht aus, einen Schwund des Materials des Zwischenelements 30, der durch Gammasterilisierung und die nachfolgende Lagerung des Zwischenelements 30 auftritt, zu kompensieren. Der maximal zu erwartende Schwund kann entweder durch ein einzelnes oder eine Mehrzahl von Ausgleichselementen 24, die umfänglich am tibiaseitigen Bereich 22 des Zwischenelements ausgeformt sind, ausgeglichen werden. Das Ausgleichselement ist bevorzugt eine vorstehende Lippe. Bei einer Mehrzahl von Ausgleichselementen kann die Höhe d der vorstehenden Lippen reduziert sein.

Zur Fixierung des Zwischenelements 30 an der Tibiakomponente 40 wird unter Kraftaufwand der Vorsprung 21 mit der vorspringenden Lippe 24 in eine Nut 25 in der Tibiakomponente 40 eingepresst, sodass sich die Lippe komprimiert. Der Vorsprung 26 in der Tibiakomponente 40 greift dabei in eine Nut 20 im Zwischenelement 30 ein. Das Zwischenelement 30 ist somit bei bereits aufgetretenem und/oder weiter zunehmendem Schwund fest und ohne Spiel in der Tibiakomponente 40 verankert. Damit bildet der femurseitige Bereich 22 des Zwischenelements 30 eine ortsfeste Artikulationsfläche.

In Fig. 2b ist ein zweites Ausführungsbeispiel des Zwischenelements 30' dargestellt. Dabei ist eine vorspringende Lippe 24' hinter dem Vorsprung 21 in der Nut 20 ausgebildet. Beim Einpassen des Zwischenelements 30' in die Tibiakomponente 40 kommt es zu einer Verpressung zwischen der vorspringenden Lippe 24' und dem Vorsprung 26 in der Tibiakomponente 40. Bei einem passgenau gefertigten Zwischenelement 30', das durch Sterilisierung mittels Gammastrahlung geschrumpft ist, reicht die Höhe der vorspringenden Lippe 24' dennoch aus, diesen Schwund zu kompensieren und ohne Spiel an dem Vorsprung 26 der Tibiakomponente 4 anzuliegen.

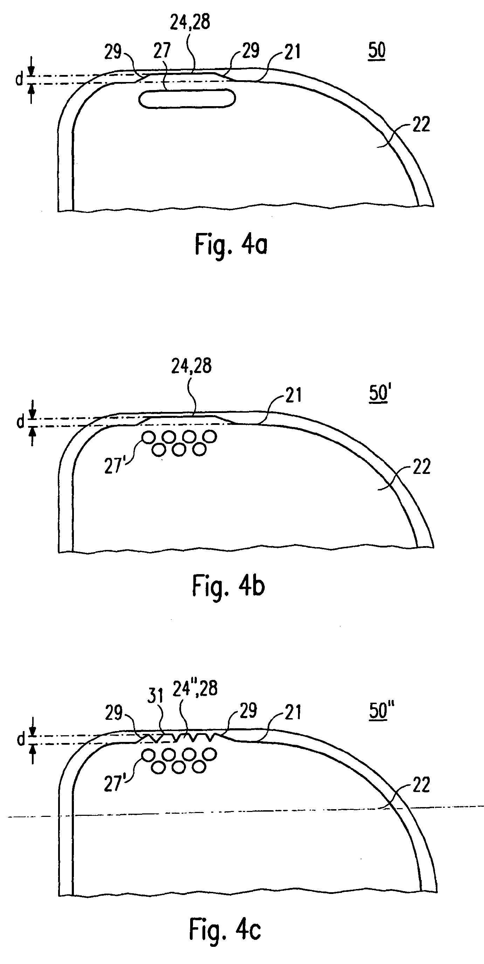

Die Figuren 4a, 4b und 4c zeigen jeweils ein Zwischenelement 50, 50'und 50'' in Draufsicht auf den tibiaseitigen Bereich 22, der eine Ausnehmung 27, 27' im Bereich des Ausgleichselements 24 aufweist. Die nachfolgend beschriebenen Ausbildungen der Ausnehmung 27, 27' und Kontur 31 der vorstehenden Lippe 28, 28' sind in gleicher Weise bei einem Ausgleichselement 24' möglich, bei dem die vorstehender Lippe 28' in der Nut 20 des Zwischenelements 30' ausgebildet ist.

In Fig. 4a ist die Ausnehmung 27 als Langloch ausgebildet und erleichtert es dem Ausgleichselement 24 sich in die Ausnehmung 27 hineinzubewegen und erleichtert somit ein Einpassen in die Tibiakomponente. Dies ist insbesondere notwendig, da der verwendete Werkstoff UHMWPE für das Zwischenelement 50 nur eine geringe Kompressibilität aufweist. Die vorspringende Lippe 28 geht mit abgeflachten Seiten 29 in den Vorsprung 21 über. Die Höhe d der vorspringenden Lippe 28 ist dabei so bemessen, dass sie einen möglichen Schwund des Materials durch die Gammasterilisation ausgleicht.

Fig. 4b zeigt ein weiteres Ausführungsbeispiel eines Zwischenelements 50', bei dem eine Mehrzahl von Ausnehmungen 27' mit kreisrunder Kontur im Bereich des Ausgleichselements 24 angeordnet sind. Andere Konturen der Ausnehmungen wie z.B. eine ovale oder eine eckige Kontur sind ebenfalls möglich.

Fig. 4c stellt ein Zwischenelement 50'' mit Ausgleichselement 24 dar, bei dem die Kontur 31 der vorstehenden Lippe 28 nicht glatt, sondern eckig ausgebildet ist. Dies ermöglicht eine Deformation der Lippe 28 in seitliche Richtung und erhöht die Kompressibilität des Ausgleichselements 24. Weist das dargestellte Ausgleichselements 24 nach anterior, so ist eine stärkere Deformation des Ausgleichselement 24 in medial-laterale Richtung durch die eckig Kontur 31 möglich. Ebenso kann die Kontur gewellt ausgeformt sein.

Alle beschriebenen und/oder gekennzeichneten Merkmale können im Rahmen der Erfindung vorteilhaft miteinander kombiniert werden. Die Erfindung ist nicht auf die gezeigten Ausführungsbeispiele beschränkt.

## Patentansprüche

1. Kniegelenk-Endoprothese, zum Ausbilden einer Gelenkverbindung zwischen einem Femurknochen und einem Tibiaknochen, mit einer Femurkomponente (2), die mit dem Femurknochen verbindbar ist, einer Tibiakomponente (4), die mit dem Tibiaknochen verbindbar ist, und mit einem zwischen der Tibiakomponente (4) und der Femurkomponente (2) angeordneten Zwischenelement (3, 30, 50),
wobei
am Zwischenelement (3, 30, 50) ein Ausgleichselement (24, 24') vorhanden ist und das Zwischenelement (3, 30, 50) mit dem Ausgleichselement (24, 24') unter einer Spannung in der Tibiakomponente (4) eingepresst ist,
**dadurch gekennzeichnet,**
**dass** das Zwischenelement (3, 30, 30',50, 50',50 ") mindestens eine Ausnehmung (27, 27') im Bereich des Ausgleichselementes (24, 24') aufweist.

2. Kniegelenk-Endoprothese nach Anspruch 1,
**dadurch gekennzeichnet,**
**dass** das Zwischenelement (3, 30, 50) auf einer an die Tibiakomponente (4) angrenzenden Seite (22) mindestens einen Vorsprung (21) aufweist, wobei der Vorsprung (21) in eine Nut (25) in der Tibiakomponente (4) eingreift und das Ausgleichselement (24) in den Vorsprung (21) integriert ist.

3. Kniegelenk-Endoprothese nach Anspruch 1,
**dadurch gekennzeichnet,**
**dass** das Zwischenelement (30') eine Nut (20) aufweist, in die ein Vorsprung (26) der Tibiakomponente (4) eingreift,
wobei das Ausgleichselement (24') im Bereich der Nut (20) im Zwischenelement (30') ausgebildet ist.

4. Kniegelenk-Endoprothese nach Anspruch 2 oder 3,
**dadurch gekennzeichnet,**
**dass** das Ausgleichselement (24, 24') als eine vorspringende Lippe (28, 28') an einem Vorsprung (21) oder im Bereich einer Nut (20) des Zwischenelements (30, 30') ausgebildet ist.

5. Kniegelenk-Endoprothese nach Anspruch 4,
**dadurch gekennzeichnet,**
**dass** die vorspringende Lippe (28, 28') mit abgeflachten Seiten (29) in den Vorsprung (21) oder in die Nut (20) des Zwischenelements (3, 30, 30', 50, 50', 50") übergeht.

6. Kniegelenk-Endoprothese nach Anspruch 4 oder 5,
**dadurch gekennzeichnet,**
**dass** die vorspringende Lippe (28, 28') eine glatte und/oder eckige und/oder gewellte Außenkontur (31) aufweist.

7. Kniegelenk-Endoprothese nach einem der Ansprüche 1 bis 6,
**dadurch gekennzeichnet,**
**dass** die Ausnehmung (27) als Langloch ausgebildet ist.

8. Kniegelenk-Endoprothese nach einem der Ansprüche 1 bis 6,
**dadurch gekennzeichnet,**
**dass** das Zwischenelement (3) eine Mehrzahl von Ausnehmungen (27') im Bereich des Ausgleichselementes (24, 24') aufweist.

9. Kniegelenk-Endoprothese nach Anspruch 6 oder 8,
**dadurch gekennzeichnet,**
**dass** die Ausnehmungen (27') mit einer kreisrunden, ovalen und/oder eckigen Kontur ausgebildet sind.

10. Kniegelenk-Endoprothese nach einem der Ansprüche 1 bis 9,
**dadurch gekennzeichnet,**
**dass** das Zwischenelement (3) aus einem Polymer besteht.

11. Kniegelenk-Endoprothese nach Anspruch 10,
**dadurch gekennzeichnet,**
**dass** das Polymer ein ultrahochmolekulares Polyethylen (UHMWPE) ist.

12. Kniegelenk-Endoprothese nach Anspruch 10 oder 11,
**dadurch gekennzeichnet,**
**dass** das Zwischenelement (3) durch Gammastrahlung sterilisiert ist.

## Claims

1. Knee joint endoprosthesis, for forming a joint connection between a femur bone and a tibia bone, with a femur component (2) which can be connected with the femur bone, a tibia component (4) which can be connected with the tibia bone, and with an intermediate element (3, 30, 50) arranged between the tibia component (4) and the femur component (2),
wherein a compensating element (24, 24') is present on the intermediate element (3, 30, 50) and the intermediate element (3, 30, 50) with the compensating element (24, 24') is pressed into the tibia component (4) under a load,
**characterised in that**
the intermediate element (3, 30, 30', 50, 50', 50") exhibits at least one recess (27, 27') in the region of the compensating element (24, 24').

2. Knee joint endoprosthesis according to claim 1,
**characterised in that**
the intermediate element (3, 30, 50) exhibits at least one projection (21) on a side (22) adjacent to the tibia component (4), wherein the projection (21) engages in a slot (25) in the tibia component (4) and the compensating element (24) is integrated in the projection (21).

3. Knee joint endoprosthesis according to claim 1,
**characterised in that**
the intermediate element (30') exhibits a slot (20) into which a projection (26) of the tibia component (4) engages, wherein the compensating element (24') is formed in the region of the slot (20) in the intermediate element (30').

4. Knee joint endoprosthesis according to claim 2 or 3,
**characterised in that**
the compensating element (24, 24') is embodied as a protruding lip (28, 28') on a projection (21) or in the region of a slot (20) of the intermediate element (30, 30').

5. Knee joint endoprosthesis according to claim 4,
**characterised in that**
the protruding lip (28, 28') runs into the projection (21) or into the slot (20) of the intermediate element (3, 30, 30', 50, 50', 50") with flattened sides (29).

6. Knee joint endoprosthesis according to claim 4 or 5,
**characterised in that**
the protruding lip (28, 28') exhibits a smooth and/or angular and/or corrugated external contour (31).

7. Knee joint endoprosthesis according to one of claims 1 to 6,
**characterised in that**
the recess (27) is embodied as an elongated slot.

8. Knee joint endoprosthesis according to one of claims 1 to 6,
**characterised in that**
the intermediate element (3) exhibits a plurality of recesses (27') in the region of the compensating element (24, 24').

9. Knee joint endoprosthesis according to claim 6 or 8,
**characterised in that**
the recesses (27') are embodied with a circular, oval and/or angular contour.

10. Knee joint endoprosthesis according to one of claims 1 to 9,
**characterised in that**
the intermediate element (3) is made of a polymer.

11. Knee joint endoprosthesis according to claim 10,
**characterised in that**
the polymer is an ultra high molecular weight polyethylene (UHMWPE).

12. Knee joint endoprosthesis according to claim 10 or 11,
**characterised in that**
the intermediate element (3) is sterilised by gamma radiation.

## Revendications

1. Endoprothèse d'articulation de genou, pour réaliser une liaison articulée entre un os de fémur et un os du tibia, comprenant un composant de fémur (2), qui peut être relié à l'os de fémur, un composant de tibia (4), qui peut être relié à l'os de tibia, et un élément intermédiaire (3, 30, 50) agencé entre le composant de tibia (4) et le composant de fémur (2),
un élément de compensation (24, 24') étant prévu sur l'élément intermédiaire (3, 30, 50), et l'élément intermédiaire (3, 30, 50) étant pressé, avec l'élément de compensation (24, 24'), sous tension ou sous contrainte dans le composant de tibia (4),
**caractérisée**
**en ce que** l'élément intermédiaire (3, 30, 30', 50, 50', 50") présente au moins un évidement (27, 27') dans la zone de l'élément de compensation (24, 24').

2. Endoprothèse d'articulation de genou selon la revendication 1,
**caractérisée**
**en ce que** l'élément intermédiaire (3, 30, 50) présente, sur un côté (22) adjacent au composant de tibia (4), au moins une protubérance (21), la protubérance (21) venant en prise dans une rainure (25) du composant de tibia (4) et l'élément de compensation (24) étant intégré à la protubérance (21).

3. Endoprothèse d'articulation de genou selon la revendication 1,
**caractérisée**
**en ce que** l'élément intermédiaire (30') présente une rainure (20) dans laquelle vient en prise une protubérance (26) du composant de tibia (4), l'élément de compensation (24') étant réalisé dans la zone de la rainure (20) dans l'élément intermédiaire (30').

4. Endoprothèse d'articulation de genou selon la revendication 2 ou la revendication 3,
**caractérisée**
**en ce que** l'élément de compensation (24, 24') est réalisé sous forme de lèvre (28, 28') en saillie sur une protubérance (21) ou dans la zone d'une rainure (20) de l'élément intermédiaire (30, 30').

5. Endoprothèse d'articulation de genou selon la revendication 4,
**caractérisée**
**en ce que** la lèvre (28, 28') en saillie se raccorde avec des côtés aplatis (29) à la protubérance (21) ou à la rainure (20) de l'élément intermédiaire (3, 30, 30', 50, 50', 50").

6. Endoprothèse d'articulation de genou selon la revendication 4 ou la revendication 5,
**caractérisée**
**en ce que** la lèvre (28, 28') en saillie présente un contour extérieur (31) lisse et/ou anguleux ou polygonal et/ou ondulé.

7. Endoprothèse d'articulation de genou selon l'une des revendications 1 à 6,
**caractérisée**
**en ce que** l'évidement (27) est réalisé sous forme de trou oblong.

8. Endoprothèse d'articulation de genou selon l'une des revendications 1 à 6,
**caractérisée**
**en ce que** l'élément intermédiaire (3) présente une pluralité d'évidements (27') dans la zone de l'élément de compensation (24, 24').

9. Endoprothèse d'articulation de genou selon la revendication 6 ou la revendication 8,
**caractérisée**
**en ce que** les évidements (27') sont formés avec un contour circulaire, ovale et/ou polygonal.

10. Endoprothèse d'articulation de genou selon l'une des revendications 1 à 9,
**caractérisée**
**en ce que** l'élément intermédiaire (3) est réalisé en un polymère.

11. Endoprothèse d'articulation de genou selon la revendication 10,
**caractérisée**
**en ce que** le polymère et un polyéthylène à ultra-haute masse moléculaire (UHMWPE).

12. Endoprothèse d'articulation de genou selon la revendication 10 ou la revendication 11,
**caractérisée**
**en ce que** l'élément intermédiaire (3) est stérilisé par rayonnement gamma.
